# EUROPEAN PATENT APPLICATION

(11) **EP 1 464 700 A1**
(43) Date of publication of application: **06.10.2004**
(21) Application number: 03701103.8
(22) Date of filing: 16.01.2003
(51) Int. Cl.: C12N 15/00, C07H 21/04, C12M 1/00, G01N 33/50

(54) **SYSTEM FOR HOUSING/PROCESSING CARRIER AND METHOD FOR HOUSING/PROCESSING CARRIER**

(30) Priority: 17.01.2002 JP 2002009218
(71) Applicant: PRECISION SYSTEM SCIENCE CO., LTD., Matsudo-shi, Chiba, 271-0064 (JP)
(72) Inventor: TAJIMA, Hideji, Precision System Science Co, Ltd., Matsudo-shi, Chiba 271-0064 (JP)
(74) Representative: Bohnenberger, Johannes, Dr.
(86) International application number: PCT/JP2003/000316
(87) International publication number: WO 2003/060115

(57) **Abstract**

An object is to provide a carrier housing/processing apparatus and a method wherein complicated reaction processes are simplified, and the processes can be easily performed with a small scale apparatus structure.

The construction comprises; one or a plurality of carriers fixed or able to be fixed with chemical substances such as ligands; a carrier housing section which is provided with a fluid inlet/outlet and which accommodates the carrier; a drawing/discharging section which draws and discharge a fluid through the inlet/outlet with respect to the carrier housing section; and a transferring section which transfers the inlet/outlet relatively with respect to containers provided outside. Moreover, the carrier is formed in a size or a shape not allowing the carrier to pass through the inlet/outlet, and in a state of holding the carrier in the housing section, by self-weight of the carrier, frictional force between the carrier and the inner wall of the housing section, or remote force from outside with respect to the carrier, a fluid is drawn and discharged.

## Description

### TECHNICAL FIELD

The present invention relates to a carrier housing/processing apparatus and its method. The present invention relates to all manner of fields which require the handling ofbiological high/low molecular weight compounds such as genes, immune systems, amino acids, proteins and sugars; including for example the fields of engineering, agricultural science incorporating foodstuffs, agricultural production and seafood processing, pharmaceuticals, medical fields incorporating hygiene, health, immunity, disease and genetics, and scientific fields such as chemistry and biology.

In particular, the present invention is an effective method in the case where a series of processes using a large number of reagents and substances are continuously executed in a predetermined order.

### BACKGROUND ART

Conventionally, in the case where a series of reaction processes using a large number of reagents and substances are executed on a target substance being a subject for examination, for example, the target substance is bonded to microcarriers such as beads and accommodated in a test tube. Then, various reagents are poured into the test tube and the carriers are separated in some way. The carriers are further transferred into another container and other reagents are poured in, or they are subjected to heat treatment. For example in the case where the carrier is a magnetic substance, the separation is performed by attaching onto the inner wall of the test tube by means to a magnetic field.

Moreover, regarding a process in which the target substance is examined using a planar carrier such as a prepared slide fixed with, for example, various oligonucleotides, by performing a series of processes such as transferring the carrier itself into a suspension having the labeled target substance suspended therein, pipetting various reagents into the carrier itself, transferring the carrier itself into a washing solution, and transferring the carrier to the measuring position of the measuring equipment so as to measure the luminescence, the base sequence structure of the target substance is examined.

In order to perform these processes, there is a problem in that the carrier itselfmust be separated and transferred, causing concern of complicated and time-consuming processes. In particular, in the case where these carriers themselves are transferred by hand, there is concern in that the user is over burdened and cross-contamination may be caused. Moreover, when the carriers themselves are transferred by machines, a large scaled device is needed. Furthermore, in the case where a non magnetic carrier is separated, it is necessary to perform the separation depending on the size and the density of the carrier, causing a problem of complicated and time-consuming processes.

On the other hand, there is a method for performing the reaction processes, not using a test tube nor a planar carrier, but using pipette device having; a pipette tip provided with a liquid passage enabling a liquid to pass therethrough, a nozzle attached with the pipette tip, a magnetic force device which exerts a magnetic field on the liquid passage of the pipette tip, and a drawing/discharging mechanism which draws a fluid into the pipette tip and discharges the fluid. According to this method, by drawing up a suspension suspending a large number of magnetic particles holding various substances on the surface, and exerting a magnetic field at the time of drawing, the magnetic particles can be effectively attached onto the liquid passage of the pipette tip so as to perform the separation.

When using this device, since the magnetic particles can pass through the liquid passage, it is necessary to apply the magnetic field to attach the magnetic particles onto the inner wall so as to hold the magnetic particles in the pipette tip. Therefore, in order to perform the processes, it is necessary to complicatedly combine the drawing/discharging control, the attraction control by the magnetic field, and the transfer control ofthe pipette tip. Moreover in the case where the carrier is a non magnetic carrier, there is a problem in that the separation can not be performed by the apparatus.

Therefore, it is a first object of the present invention to provide a carrier housing/processing apparatus and a method wherein, regarding a carrier which is fixed or able to be fixed with various substances, by enabling processes to be performed while accommodating and holding in the carrier housing section, the attraction control for accommodating and holding the carrier in the housing section and the drawing control, are made unnecessary, so that the complicated reaction processes are simplified, and the processes can be easily performed with a small scale apparatus structure.

It is a second object to provide a carrier housing/processing apparatus and a method wherein, regarding a carrier which is fixed or able to be fixed with various substances, by accommodating and removing these substances by a route which is different from a route for drawing/discharging a fluid or substances suspended in the fluid, the process for separating the fluid and the carrier become unnecessary, so that the complicated reaction processes are simplified, and the processes can be easily performed with a small scale apparatus structure.

It is a third object to provide a carrier housing/processing apparatus and a method wherein, regarding a carrier which is fixed or enable to be fixed with various substances, by enabling easy removal of the substances from the carrier housing section accommodating them, it becomes possible to easily exchange and preserve the carrier or perform other processes with respect to the carrier so that the efficiency, the reliability and the certainty of processes can be increased.

It is a fourth object to provide a carrier housing/processing apparatus and a method wherein, by appropriately determining the shape and the size of carrier so as to not limit the material of the magnetic substance, regardless of the material of the carrier, it becomes possible to easily separate, so that the selection range for the materials can be increased, and the most suitable material for a process can be selected.

### DISCLOSURE OF THE INVENTION

In order to address the above technical problems, a first aspect of the invention is a carrier housing/processing apparatus comprising; one or a plurality of carriers fixed or able to be fixed with chemical substances such as ligands; a carrier housing section which is provided with a fluid inlet/outlet and which accommodates the carrier; a drawing/discharging section which draws and discharge a fluid through the inlet/outlet with respect to the carrier housing section; and a transferring section which transfers the inlet/outlet relatively with respect to containers provided outside, wherein the carrier is formed in a size or a shape not allowing the carrier to pass through the inlet/outlet, and in a state ofholding the carrier in the housing section, by self-weight of the carrier, frictional force between the carrier and the inner wall of the housing section, or remote force from outside with respect to the carrier, a fluid is drawn and discharged.

Here, "ligand" includes chemical substances including living bodies of biological high/low molecular weight compounds, such as for example, genetic substances such as nucleic acid, proteins, sugars, sugar chains, peptides, and the like. The ligand is used as a detection substance which detects the bond ofa receptor having bondability with the ligand to capture, separate, and extract. Receptor includes chemical substances of biological high/low molecular weight compounds, such as; genetic substances such as nucleic acid, proteins, sugar chains, peptides, and the like, having bondability with each of the genetic substances such as nucleic acid, proteins, sugar chains, peptides, and the like. 'Fixing' includes the cases of, for example, physical attachment, electrical interaction, and the like, as well as the cases of covalent bond and chemical absorption.

The carrier is a member which is fixed or able to be fixed with chemical substances such as one or more kinds of ligands, and held by "self-weight of the carrier, frictional force with the inner wall of the housing section, or remote force from outside with respect to the carrier" in the carrier housing section. As a result, the holding state can be easily released by lifting the carrier by a greater force than the self-weight of the carrier, moving by a force against the frictional force, or removing the remote force.

In this case, by providing an opening having a size enabling the carrier to pass through the carrier housing section but the opening not allowing the fluid to pass through, it becomes possible to exchange, remove, and preserve only the carrier in the carrier housing section. The carrier may be in the state of being bonded with predetermined ligands or receptors in some cases, or in the state of being bonded with no substance in other cases. The carrier removed, preserved, and exchanged from the carrier housing section may further be an object of a process in other processes. Consequently, various processes become possible enabling diversification of the processes.

In the case where the magnetic field is used as the remote force for example, as a prerequisite, it is necessary that the carrier itself is a magnetic substance or includes a magnetic substance, and outside of the carrier housing section there is a magnetic force device which exerts a magnetic field on the carrier housing section interior. In the case where an electric field is used, it is necessary that the carrier is a charged object carrying an electric charge. Moreover, the carrier is fixed with the chemical substances by chemical adsorption, physical adsorption, specific reaction with bonding substances provided fixed to the carrier, and other ways. Moreover, the carrier may be formed from a porous member, a corrugated surface member, or a fibrous member so as to increase the reaction ability and the bondability with the chemical substances.

The carrier is preferably held by self-weight, frictional force, or remote force to a degree which keeps the carrier from floating up or being moved by the presence of fluid in the carrier housing section, or by the drawing/discharging of fluid into/out of the carrier housing section, so that the fluid can contact sufficiently with the carrier according to the amount of the fluid. Particularly in the case where the ligand or the like is fixed to the carrier in a position according its chemical structure in order to measure the luminescence, the position of the carrier is preferably unchanged.

According to this aspect of the invention, regarding a carrier which is fixed or able to be fixed with various substances, the arrangement is such as to enable processes to be performed while accommodated in the carrier housing section. Therefore, the attraction control or the drawing control for accommodating and holding the carrier in the housing section become unnecessary, so that the complicated reaction processes are simplified, and the processes can be easily performed with a small scale apparatus structure.

Moreover, according to this aspect of the invention, regarding a carrier which is fixed or able to be fixed with various substances, the arrangement is to accommodate and remove these substances by a route which is different from a route for drawing/discharging a fluid or substances suspended in the fluid. Therefore, the process for separating the fluid and the carrier becomes unnecessary, so that the complicated reaction processes are simplified and the processes can be easily performed with a small scale apparatus structure.

Furthermore, according to this aspect of the invention, regarding a carrier which is fixed or able to be fixed with various substances, the arrangement is such as to enable easy removal of the substances from the carrier housing section accommodating them. Therefore, it becomes possible to easily exchange and preserve the carrier or perform other processes with respect to the carrier, so that the efficiency, the reliability and the certainty ofprocesses can be increased.

Moreover, according to this aspect of the invention, only by drawing/discharging a fluid while holding the carrier in the carrier housing section and transferring the carrier housing section, various processes; for example, reacting, washing, temperature control, separating, mixing, pipetting, clarifying, isolating, eluting, and extracting can be performed, so that the processes can be effectively, rapidly and easily performed.

A second aspect of the invention is a carrier housing/processing apparatus wherein the carrier housing section has a large diameter section which accommodates the carrier and a small diameter section which has the inlet/outlet at the tip and has a smaller diameter enabling insertion into containers provided outside.

Furthermore, the arrangement may be such that the carrier housing section has a large diameter section, a small diameter section which has a smaller diameter than the large diameter section, and an intermediate diameter section which is provided between the large diameter section and the small diameter section having a diameter intermediate between them In this case, the arrangement is such that the intermediate diameter section accommodates the carrier. In this case, the arrangement is such that, by forming the intermediate diameter section into a size capable of accommodating the carrier but making the volume ofthe rest of the space in the intermediate diameter section in the case of accommodating the carrier sufficiently smaller than the volume of the carrier, the processes can be performed on a fluid which has a sufficiently smaller volume of the fluid being drawn than the volume of the carrier. Moreover, by setting the volume of the large diameter section to the maximum volume of the container which accommodates the liquid being the object of the process, it becomes possible to also cope with a process which manages liquid ofthe maximum volume capable of being accommodated in the container. The boundary of the small diameter section, the intermediate diameter section, and the large diameter section may be formed so as not to rapidly change in diameter, but to gradually change from the large diameter to the small diameter.

According to this aspect of the invention, by providing the small diameter section which is capable of insertion into external containers, the housing apparatus may be transferred between containers including containers set in a constant temperature, while accommodating the carrier to draw/discharge liquids such as various reagents, suspensions, and the like accommodated in the respective containers, so that reacting, washing, temperature control, separating, mixing, pipetting, clarifying, isolating, eluting, and extracting can be performed. Therefore, a series ofprocesses may be reduced to the transferring process between containers so as to simplify the control.

A third aspect of the invention is a carrier housing/processing apparatus wherein the carrier housing section has an opening having a size enabling the carrier to pass through, and the drawing/discharging section is provided with a nozzle which detachably connects with the opening, and the carrier is formed in a size capable of passing through the opening but not capable of passing through the inlet/outlet.

Here, the carrier may be previously accommodated in the housing section prior to installation onto the nozzle. In this case, the opening may be detachably covered by a lid body so as to enclose the carrier.

According to this aspect of the invention, since the carrier is accommodated into the carrier housing section so as to be able to be taken out, then by exchanging the carrier, it becomes possible to reliably prevent cross-contamination, and to preserve the carrier, and to perform further processes.

A fourth aspect of the invention is a carrier housing/processing apparatus wherein the carrier is; a particle having a larger diameter than the inlet/outlet, a block member having a shape not capable of passing through the inlet/outlet, a sheet member, a wire like member formed by bending in a predetermined size, or an indeterminate member. Here, block-like member includes spherical, cylindrical, and prismatic members.

According to this aspect of the invention, various materials can be used so as to diversify the processes.

A fifth aspect of the invention is a carrier housing/processing apparatus wherein the plurality of carriers are a plurality of kinds.

According to the this aspect of the invention, by accommodating a plurality of carriers, a plurality of processes may be performed in parallel so as to perform the processes effectively, rapidly and easily.

A sixth aspect of the invention is a carrier housing/processing apparatus wherein the carrier is provided with an adhesion prevention section such as a projection, a ditch, a corrugated surface, for keeping the carrier from being adhered to the inner wall of the carrier housing section.

According to this aspect of the invention, by providing the adhesion prevention section such as a projection, a ditch, a corrugated surface for keeping the carrier from being adhered to the inner wall of the carrier housing section, the contact efficiency of the carrier and a fluid can be increased.

A seventh aspect of the invention is a carrier housing/processing apparatus wherein the carrier housing section is provided with an adhesion prevention section such as a projection, a ditch, a corrugated surface, for keeping from being adhered to the carrier.

According to this aspect of the invention, by providing the adhesion prevention section such as a projection, a ditch, a corrugated surface for keeping the carrier from being adhered with the inner wall of the carrier housing section, the contact efficiency of the carrier and a fluid can be increased.

An eighth aspect of the invention is a carrier housing/processing apparatus wherein the carrier is; a member having through holes, a permeable membrane member, a porous member, or a mesh member, which is held at a predetermined position in the carrier housing section so as to divide and partition the carrier housing section into upper and lower spaces, and allow a fluid to pass therethrough.

According to this aspect of the invention, since the carrier is provided so that the housing section is divided and separated into upper and lower spaces, and a fluid can pass through, the contact efficiency with the fluid is high.

A ninth aspect of the invention is a carrier housing/processing apparatus wherein the carrier is held at the bottom of the carrier housing section, which is a predetermined position, by self-weight, and carrier holding sections such as projections, ditches, corrugated surfaces are provided at the bottom so as to keep the carrier from blocking passage of the fluid.

According to this aspect of the invention, since carrier holding sections are provided at the bottom so as not to block passage of a fluid, the carrier and the fluid can be reliably contacted to increase the efficiency of the process.

A tenth aspect of the invention is a carrier housing/processing apparatus wherein the carrier housing section is formed from a translucent member, and a measuring apparatus which measures luminescence on the carrier is provided outside of the carrier housing section.

According to this aspect of the invention, since the measuring apparatus which measures the luminescence is provided, not only reaction but also processes including measuring may be consistently performed.

An eleventh aspect of the invention is a carrier housing/processing apparatus wherein in the carrier housing section, a side face provided with the measuring equipment is formed in a plane. Accordingly, the luminescence may be reliably measured.

According to this aspect of the invention, the measurement ofthe luminescence may be reliably performed.

A twelfth aspect of the invention is a carrier housing/processing apparatus wherein the carrier contains a magnetic substance and the carrier is held in a predetermined position of the carrier housing section due to a magnetic field exerted from outside of the carrier housing section.

According to this aspect of the invention, by holding the carrier in a predetermined position of the housing section due to the magnetic substance, the luminescence including position may be reliably measured.

A thirteenth aspect of the invention is a carrier housing/processing apparatus wherein the carrier is a glass or its surface is coated with a glass.

According to this aspect of the invention, by using a carrier coated with a glass on the surface, DNA substances can be easily captured.

A fourteenth aspect of the invention is a carrier housing/processing method comprising; a drawing/contacting step for, with respect to a housing section which accommodates one or a plurality of carriers fixed or able to be fixed with chemical substances such as ligands and has an inlet/outlet which enables a fluid to pass through but does not enable the carriers to pass through, drawing fluid through the inlet/outlet from external containers by a drawing/discharging section, to contact the carriers which are held in the carrier housing section by self-weight of the carrier, frictional force with the inner wall of the carrier housing section, or remote force from outside with respect to the carrier, with the drawn fluid; and a discharging step for discharging only the fluid through the inlet/outlet by the drawing/discharging section, in a state where the carrier is accommodated in the carrier housing section.

This aspect of the invention has a similar effect to the effect described in the first aspect of the invention.

A fifteenth aspect of the invention is a carrier housing/processing method further comprising a transferring step for transferring the inlet/outlet relatively with respect to containers provided outside.

According to this aspect of the invention, various processes may be performed by transferring the inlet/outlet between containers.

A sixteenth aspect of the invention is a carrier housing/processing method wherein the reaction step has a step for repeatedly drawing and discharging a fluid with respect to the carrier housing section.

According this aspect of the invention, by repeatedly drawing and discharging a fluid with respect to the carrier housing section, the reaction can be performed effectively and reliably.

A seventeenth aspect of the invention is a carrier housing/processing method further comprising an accommodating step for accommodating the carrier in the carrier housing section from an opening having a size enabling the carrier to pass through.

According to this aspect of the invention, since the carrier is accommodated in the carrier housing section so as to be able to be taken out, then by exchanging the carrier or the like, it becomes possible to reliably prevent cross-contamination, and to preserve the carrier, and to perform further processes.

An eighteenth aspect of the invention is a carrier housing/processing method further comprising a removing step for removing the carrier from the carrier housing section through an opening having a size enabling the carrier provided in the carrier housing section to pass through.

According to this aspect of the invention, since the carrier is accommodated in the carrier housing section so as to be able to be taken out, then by exchanging the carrier or the like, it becomes possible to reliably prevent cross-contamination, to preserve the carrier, and to perform further processes.

### BRIEF DESCRIPTION OF THE DRAWINGS

FIG. 1 is a schematic illustration of a carrier housing reactor according to the first embodiment of the present invention.
FIG. 2 is a schematic illustration of carrier housing reactors of according to the second and the third embodiments of the present invention.
FIG. 3 is a perspective illustration of a carrier housing reactor of according to the forth embodiment of the present invention.
FIG. 4 shows an example of a process using the carrier housing reactor according to the first embodiment of the present invention.

### BEST MODE FOR CARRYING OUT THE INVENTION

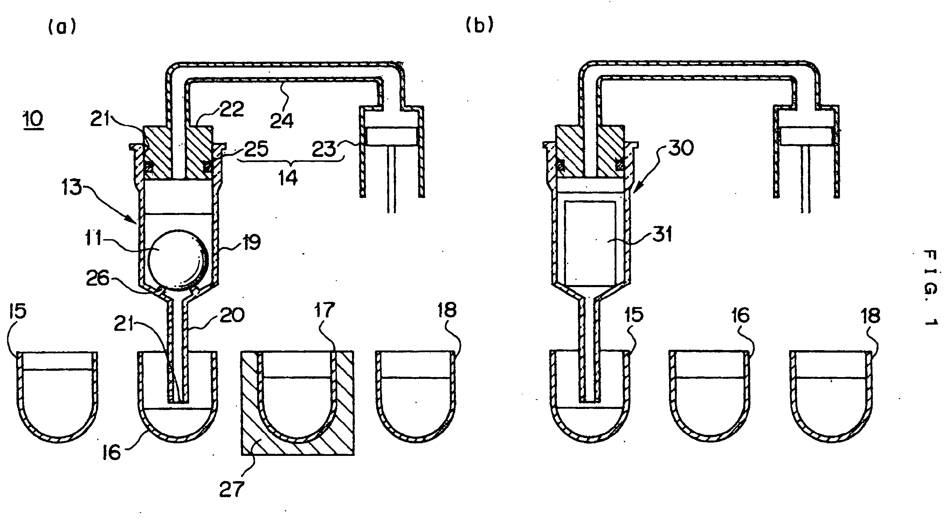

Next is a description of, a carrier housing/processing apparatus 10 according to a first embodiment of the present invention, based on FIG. 1. The description of the respective embodiments should not be considered as limiting the present invention, unless particularly specified.

As shown in FIG. 1 (a), the carrier housing/processing apparatus 10 according to the present embodiment has one spherical carrier 11 capable fixing DNA having a predetermined base sequence as a ligand on the surface, and a fluid inlet/outlet 12 for, and is provided with an approximate cylindrical carrier housing section 13 accommodating the spherical carrier 11, a drawing/discharging section 14 which draws and discharges a fluid with respect to the carrier housing section 13 through the inlet/outlet 12, and a transferring section (not shown) which relatively transfers the fluid inlet/outlet 12 of the carrier housing section 13, with respect to containers 15 to 18 externally provided.

Moreover, the carrier housing section 13 has a large diameter section 19 which accommodates the spherical carrier 11, and a small diameter section 20 which has the inlet/outlet 12 at the tip and has a smaller diameter than the large diameter section, enabling insertion into the containers 15 to 18. The diameter of the spherical carrier 11 is formed larger than the internal diameter of the small diameter section 20, that is the bore of the inlet/outlet 12, so as to keep the spherical carrier 11 from passing through the small diameter section 20 and being discharged out of the carrier housing section 13. Furthermore, the carrier housing section 13 has an opening 21 having a size enabling the spherical carrier 11 to pass through.

The drawing/discharging section 14 has; a nozzle section 22 which detachably connects with the opening 21 at the tip, a cylinder 23, and a thin pipe 24 which connects the cylinder 23 and the nozzle section 22. Furthermore, an O-ring 25 for maintaining watertightness, is provided between the nozzle section 22 and the carrier housing section 13.

At the bottom of the large diameter section 19 in the carrier housing section 13, there are provided carrier holding sections 26 protruding upwards so as to hold the spherical carrier 11. The carrier holding sections 26 are provided arranged for example at three or four places at equal intervals around the circumference of the hole in the center of the bottom ofthe large diameter section 19.

The containers 15 to 18 accommodate specimens, reagents and the like required according to the sequence of the processes. The container 17 is accommodated in a thermostatic block 27 which keeps a predetermined temperature so as to maintain a liquid accommodated in the container 17 at the predetermined temperature.

To the spherical carrier 11 is fixed or able to be fixed, a predetermined ligand, for example, a single strand DNA. The spherical carrier 11 itself is formed in a porous shape, increasing the holding ability of the ligand and enabling a liquid to pass through inside so as to increase the possibility that the ligand and the liquid encounter. The spherical carrier 11 is formed so as to have a density sufficiently larger than the density of a liquid drawn and discharged with respect to the carrier housing section 13, and is mounted on the carrier holding sections 26 being the protruding members, by self-weight.

The spherical carrier 11 may be formed from for example, a resin, a glass, a ceramic, a metal, and the like, including nylon, polyethylene, polyester, polypropylene, urethane, gum, and the like. Moreover, the materials may be combined so as to have the self-weight, by placing a weight formed from a metal inside, and forming the exterior from a resin.

FIG. 1(b) shows a carrier housing/processing apparatus according to another embodiment.

The apparatus differs from the carrier housing/processing apparatus 10 according to the first embodiment, in that a sheet carrier 31 is provided instead of the spherical carrier 11 inside a carrier housing section. The dimensions of at least two of the width, the length, and the thickness of the sheet carrier 31 are formed larger than the internal diameter of the small diameter section 20, that is the bore of the inlet/outlet 12, so as to keep the sheet carrier 31 from passing through the small diameter section 20 and being discharged out of the carrier housing section 30.

Moreover, the carrier housing section 30 has an opening 21 having a size enabling the sheet carrier 31 to pass through. Due to the shape ofthe sheet carrier 31, the sheet carrier 31 does not clog in the passage of the small diameter section 20 leading to the inlet/outlet. Therefore, the carrier holding section 26 is not provided. The material of the sheet carrier 31 is similar to the case of the abovementioned spherical carrier 11.

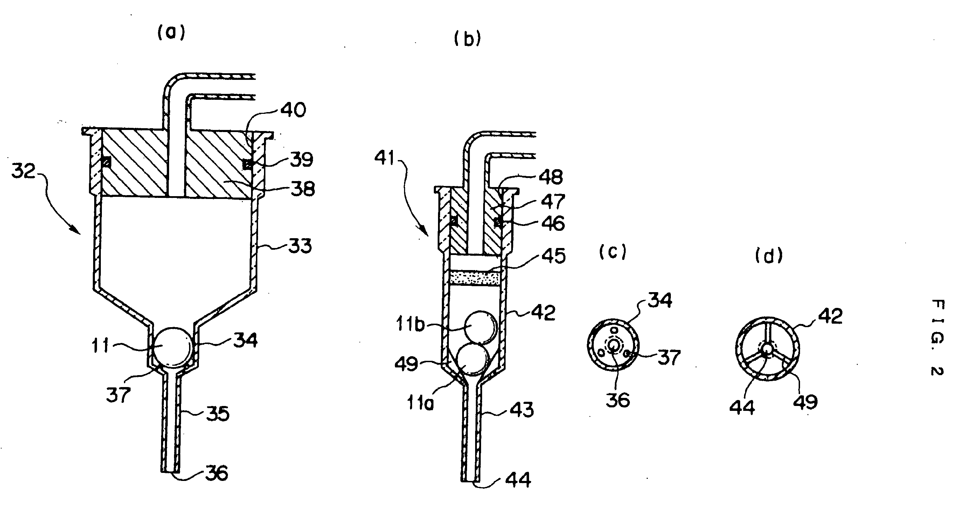

FIG. 2(a) and (c) show an example of a carrier housing section 30 according to a second embodiment.

The carrier housing section 30 is approximate cylindrical having approximate three steps accommodating the spherical carrier 11 inside. The carrier housing section 30 has a fluid inlet/outlet 36, and is fitted to a nozzle section 38 of a drawing/discharging section which draws and discharges a fluid with respect to the carrier housing section 30, passing through the inlet/outlet 36. Moreover it is possible to transfer the inlet/outlet 36 of the carrier housing section 30 relatively with respect to a container (not shown) provided outside, by a transferring section (not shown).

The carrier housing section 30 has a large diameter section 33 which is able to accommodate a fluid, with its opening 40 fitted to the nozzle section 38 via an O-ring 39, a small diameter section 35 which has the inlet/outlet 36 at the tip and has a smaller diameter than the large diameter section enabling insertion into the containers, and an intermediate diameter section 34 which is provided between the large diameter section 33 and the small diameter section 35, having a diameter intermediate between them, and which accommodates the spherical carrier 11.

Moreover, the diameter of the opening 40 of the large diameter section 33 is formed larger than the spherical carrier 11, enabling the spherical carrier 11 to pass through. According to the present embodiment, the reaction process may be performed with the degree of the volume of the rest of the space in the intermediate diameter section 34 accommodating the spherical carrier 11, that is the small amount of fluid having a sufficiently smaller volume than the volume of the spherical carrier 11. At the bottom ofthe intermediate diameter section 34, as shown in FIG. 2 (c), there are provided carrier holding sections 37, protruding upwards at a plurality of places so as to hold the spherical carrier 11.

FIG. 2 (b) and (d) show an example of a carrier housing section 41 according to the third embodiment.

The carrier housing section 41 is approximate cylindrical having substantially two steps accommodating two spherical carriers 11a and 11b. The carrier housing section 41 has a fluid inlet/outlet 44 and is fitted to a nozzle section 47 of a drawing/discharging section which draws and discharges a fluid with respect to the carrier housing section 41, passing through the inlet/outlet 41. Moreover it is possible to transfer the inlet/outlet 44 ofthe carrier housing section 41 relatively with respect to a container (not shown) provided outside, by a transferring section (not shown).

The carrier housing section 41 has a large diameter section 42 which is able to accommodate a fluid, with its opening 48 fitted to the nozzle section 47 via an O-ring 46, and a small diameter section 43 which has the inlet/outlet 44 at the tip and has a smaller diameter than the large diameter section enabling insertion into the containers. Moreover, in the large diameter section 42, a filter 45 enabling only air to pass through, is provided so as to divide the large diameter section into two upper and lower spaces. Accordingly, a liquid drawn into the lower space can be kept from invading into the upper space, so as to reliably prevent cross-contamination due to contamination of the nozzle section 47 itself.

Moreover, at the bottom of the large diameter section 42, approximate triangular carrier holding sections 49 which hold the spherical carrier 11a without blocking the flow of fluid, are radially provided rising up from the vicinity of the center along the radial direction.

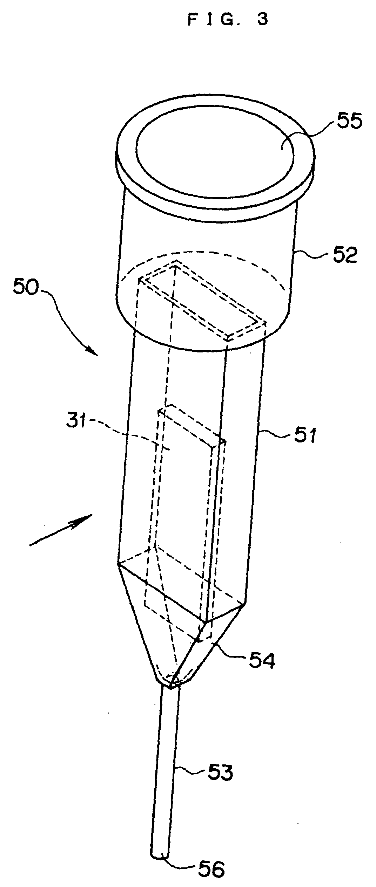

FIG. 3 shows a carrier housing section 50 according to a fourth embodiment.

The carrier housing section 50 has an attachment section 52 which is able to accommodate a fluid, with its opening 55 fitted to the cylindrical nozzle section (not shown) via an O-ring, a measurement housing section 51 which communicates with the attachment section 52, formed from an approximate prismatic transparent or semitransparent member capable of accommodating a sheet carrier 31 inside, an intermediate section 54 which is approximate pyramidal and communicates with the measurement housing section 51, and a small diameter section 53 which is provided on the intermediate section 54, formed thinner than the large diameter section enabling insertion into containers provided outside, and having a fluid inlet/outlet 56 at the tip. Moreover, it is possible to transfer the inlet/outlet 56, that is the carrier housing section 50 relatively with respect to containers provided outside, by a transferring section.

The intermediate section 54 plays a role of a carrier holding section which holds the aforementioned sheet carrier 31. On the side face of the measurement housing section 51 is provided a luminescent section or a photoreceptive section of a photometer, enabling measurement of the luminescence from the sheet carrier 31 accommodated in the measurement housing section 51, which transmitting orthogonally to the side face. Accordingly, the measurement can be performed more certainly and reliably.

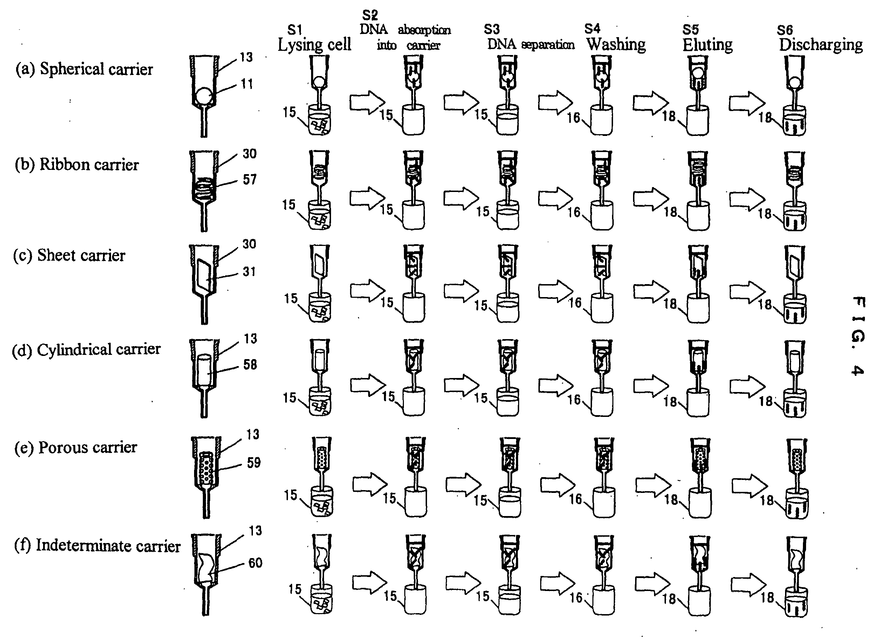

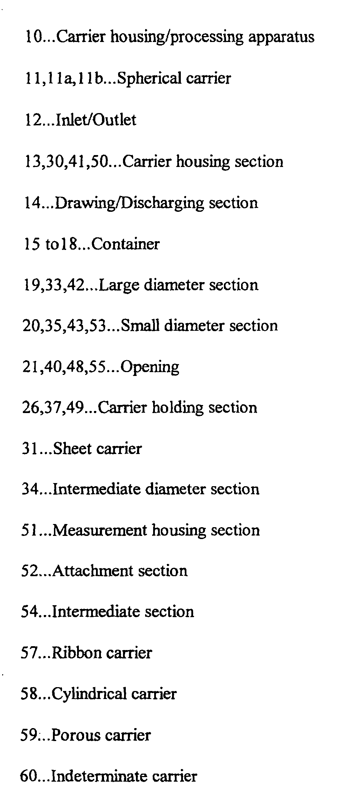

FIG. 4 illustrates a case where a process is performed in which DNA is extracted from cells using a carrier housing/processing apparatus having carrier housing sections 13 and 30 accommodating various carriers 11, 31, 57, 58, 59, and 60.

FIG. 4 (a) shows a case where the spherical carrier 11 is accommodated in the carrier housing section 13 as described above. FIG. 4 (b) shows a case where a ribbon carrier 57 is accommodated in the carrier housing section 30. The ribbon carrier 57 is a wire like member which is not deformed unless a certain degree of force is applied, and is bent in a shape so as not to pass through the small diameter section 20, that is the inlet/outlet 12, and is held in the carrier housing section 30 by self-weight.

FIG. 4 (c) shows a case where the sheet carrier 31 is accommodated in the carrier housing section 30. The sheet carrier 31 is the same as described in FIG. 1.

On the other hand, FIG. 4 (d) shows a case where a cylindrical carrier 58 is accommodated in the carrier housing section 13. The diameter ofthe cylindrical carrier 5 8 is formed larger than the internal diameter of the small diameter section 20, that is the bore of the inlet/outlet 12, so as to keep the cylindrical carrier 58 from passing through the small diameter section 20 and being discharged out of the carrier housing section 13. The cylindrical carrier 58 is held in the carrier housing section 30 by self-weight, or by frictional force with the inner wall of the carrier housing section 13. The material of the cylindrical carrier 58 is similar to the material described for the spherical carrier 11.

FIG. 4 (e) shows a case where the porous carrier 59 is accommodated in the carrier housing section 13. The porous carrier 59 is formed from a porous material and provided with a large number of through holes enabling a liquid to pass through inside. Here, an example is shown where the porous carrier 59 is cylindrical. The diameter is formed larger than the internal diameter of the small diameter section 20, that is the bore ofthe inlet/outlet 12. Accordingly, the surface area is increased so that the capturing ability for bonding a target substance can be increased.

Furthermore, FIG. 4 (f) shows a case where an indeterminate carrier 60 is accommodated in the carrier housing section 13. In this case, the diameter of the indeterminate carrier 60 has a shape or a size which is unable to pass through the small diameter section 20, that is the inlet/outlet 12, so as to keep the indeterminate carrier 60 from passing through the small diameter section 20 and being discharged out of the carrier housing section 13. The indeterminate carrier 60 is held in the carrier housing section 30 by self-weight, or by frictional force with the inner wall of the carrier housing section 13.

The case of FIG. 4 (a) is described.

In step S1, for example, in order to destruct cells such as microbial bodies, a cell lysate solubilized with high concentration guanidine isothiocyanate (protein denaturant) or surfactant, to bare DNA closed in each cell, is accommodated in a container 15.

Moreover, in order to capture the DNA in the lysate, the spherical carrier 11 coated with a material capable of bonding the DNA, for example a glass or a silica gel, is inserted into the carrier housing section 13 from the opening 21 and accommodated therein.

In step S2, the small diameter section 20 ofthe carrier housing section 13 is relatively transferred to the container 15 accommodating the cell lysate in which the cells were lysed, and is inserted into the container 15. Then, the lysate in the container 15 is drawn into the carrier housing section 13 accommodating the spherical carrier 11, by the drawing/discharging section 14 until the spherical carrier 11 is completely immersed in the lysate, and is then discharged. This action is repeated several times. As a result, the spherical carrier 11 accommodated in the carrier housing section 13 and the lysate contact with each other so that the DNA in the lysate is absorbed into the spherical carrier 11. Since it is under the presence of thiocyanate ions (also called chaotropic ions or chaotrope ions) which play a role to dehydrate the hydrated water in the lysate, DNA is in the state of being absorbed easily into the silica surface. The reason why it is in the state of being absorbed easily, is generally that since the absorption ability of silica gel decreases according to the amount of hydrated water, the absorption ability increases by capturing the hydrated water.

In step S3, the residual liquid of the lysate is discharged from the carrier housing section 13 into the container 15. Then in a state with the lysate discharged from the carrier housing section 13 and the DNA separated by the spherical carrier 11, the carrier housing section 13 is transferred relatively with respect to a region where various containers are mounted, to a container 16 accommodating a washing reagent. In this separation state, proteins as well as the DNA are not completely removed, and are in the absorbed state.

In step S4, the small diameter section 20 is inserted into the container 16, and a protein denaturant having a similar function as in step S1, and an ethanol which are washing reagents, are used so as to perform washing which removes the proteins without removing the DNA from the spherical carrier 11. That is, the proteins are denatured by the protein denaturant so as to destroy the proteins, and hydration with DNA is prevented by the ethanol so as to generate precipitation. In this case, since the ethanol is 70% concentration, components having the function of generating precipitation and contained as the absorption liquid and the washing solution are desalinized, and the solutions are replaced by solutions of low salt concentration. As a result, the hydration with the DNA is prevented at the same time.

In step S5, in a state with the spherical carrier 11 absorbing the washed DNA accommodated therein, the carrier housing section 13 is further transferred relatively with respect to the region where various containers are mounted, to a container 18 accommodating an eluate. The small diameter section 20 is then inserted into the container 18, and the drawing and the discharging are repeated so as to elute the DNA captured by the spherical carrier 11 into the eluate. Here, the eluate is performed, for example using miliQ or TE (Tris-EDTA) buffer solution. Then, the DNA dehydrated in step S4 is re-hydrated and dissolved. At the same time, the silica surface becomes a state where there is no chaotrope ions and is easier to be hydrated. Since originally the interaction between the DNA and the silica is more than a little, it is more effective if the elution is performed while increasing the temperature, or mixing.

In step S6, all of the liquid in the carrier housing section 13 is discharged into the container 18 so as to obtain the DNA.

Instead of following the elution processes of step S5 and step S6, the DNA may be taken out from the carrier housing section 13 while bonded with the spherical carrier 11, to use for other processes and the like.

The above described respective embodiments have been described in detail in order to better understand the present invention, and in no way limit other aspects. Consequently, the embodiments can be modified within a scope which does not alter the gist of the invention. For example, in the embodiments, only the case of the spherical carrier 11 was described in detail. However the present invention is not limited to this case and is also applicable to the ribbon carrier 57 such as a wire like member, the sheet carrier 31, the cylindrical carrier 58, the porous carrier 59, and the indeterminate carrier 60.

Furthermore, in the present invention, even if the member is a flexible slender member such as ribbon or string which is fixed or able to be fixed with chemical substances such as one or more kinds of ligands on the side face thereof, as long as it is an integrated slender member which is coiled using or not using a supporting body such as a cylindrical core or the like, and integrated by lamination or alignment, and is formed in a size or a shape which is unable to pass through the inlet/outlet, and which allows drawing and discharging of a fluid while being held in the carrier housing section, it may be used as the carrier.

In the case of a flexible slender member or a wire like member, by fixing or being able to be fixed with the chemical substances including living bodies along the circumference direction orthogonal to the longitudinal direction, the chemical substances can be accurately and easily arranged in the same manner on a side face viewed from any direction. Therefore, a reaction or a measurement process may be performed on the side face viewed from any direction. Furthermore, by lowering the density of chemical substances including ligands with respect to all side faces of the slender member, manufacture can be easier and reliability can be increased. On the other hand, at the time of processing, by accommodating the integrated slender member, processing efficiency can be increased. Moreover, at the time of measuring, since the arrangement of chemical substances can be reliably corresponded along a one-dimensional path, measurement reliability is high. The flexible slender member includes for example a member formed from a chemical fiber such as nylon or the like.

The carrier includes a member having one or more kinds of chemical compounds arranged at intervals, so that their positions can be specified, and a member having one or more kinds of chemical compounds not arranged so that their positions can be specified, but fixed or able to be fixed.

Moreover, the abovementioned respective components, the respective carriers, the respective carrier housing sections, the carrier holding sections, the containers, the drawing/discharging sections, the small diameter sections, the large diameter sections, the intermediate diameter section, the intermediate section, the nozzle sections, and the like, or the respective apparatus may be arbitrarily combined with appropriate modification.

Furthermore, in the abovementioned examples, only the case of DNA extraction was described. However the carrier may be fixed with ligands or the like in order to detect a target substance. Moreover, the ligand is not limited to DNA but includes genetic substances such as oligonucleotides and RNA, immunity substances, proteins, sugar chains, and further includes pheromones, allomones, mitochondorias, virus, plasmids, and the like.

Moreover, the abovementioned reagents or substances show one example, and other reagents or substances may be used. Furthermore, a carrier capturing DNA or the like may be taken out from the carrier housing section and preserved, or made an object of other processes.

## Claims

1. A carrier housing/processing apparatus comprising;
one or a plurality of carriers fixed or able to be fixed with chemical substances such as ligands; a carrier housing section which is provided with a fluid inlet/outlet and which accommodates said carrier; a drawing/discharging section which draws and discharge a fluid through said inlet/outlet with respect to said carrier housing section; and a transferring section which transfers said inlet/outlet relatively with respect to containers provided outside, wherein
said carrier is formed in a size or a shape not allowing said carrier to pass through said inlet/outlet, and in a state of holding said carrier in said housing section, by self-weight of said carrier, frictional force between said carrier and the inner wall of said housing section, or remote force from outside with respect to said carrier, a fluid is drawn and discharged.

2. A carrier housing/processing apparatus according to claim 1, wherein said carrier housing section has a large diameter section which accommodates said carrier and a small diameter section which has said inlet/outlet at the tip and has a smaller diameter enabling insertion into containers provided outside.

3. A carrier housing/processing apparatus according to claim 1, wherein said carrier housing section has an opening having a size enabling said carrier to pass through, and said drawing/discharging section is provided with a nozzle which detachably connects with said opening,
and said carrier is formed in a size capable of passing through said opening but not capable of passing through said inlet/outlet.

4. A carrier housing/processing apparatus according to either one of claim 1 and claim 3, wherein said carrier is; a particle having a larger diameter than said inlet/outlet, a block member having a shape not capable of passing through said inlet/outlet, a sheet member, a wire like member formed by bending in a predetermined size, or an indeterminate member.

5. A carrier housing/processing apparatus according to any one of claim 1 through claim 4, wherein said plurality of carriers are a plurality of kinds.

6. A carrier housing/processing apparatus according to any one of claim 1 through claim 5, wherein said carrier is provided with an adhesion prevention section such as a projection, a ditch, a corrugated surface, for keeping said carrier from being adhered to the inner wall of said carrier housing section.

7. A carrier housing/processing apparatus according to any one of claim 1 through claim 6, wherein said carrier housing section is provided with an adhesion prevention section such as a projection, a ditch, a corrugated surface, for keeping from being adhered to said carrier.

8. An carrier housing/processing apparatus according to any one of claim 1 through claim 7, wherein said carrier is; a member having through holes, a permeable membrane member, a porous member, or a mesh member, which is held at a predetermined position in said carrier housing section so as to divide and partition said carrier housing section into upper and lower spaces, and allow a fluid to pass therethrough.

9. A carrier housing/processing apparatus according to any one of claim 1 through claim 8, wherein said carrier is held at the bottom of said carrier housing section, which is a predetermined position, by self-weight, and carrier holding sections such as projections, ditches, corrugated surfaces are provided at the bottom so as to keep said carrier from blocking passage of said fluid.

10. A carrier housing/processing apparatus according to any one of claim 1 through claim 9, wherein said carrier housing section is formed from a translucent member, and a measuring apparatus which measures luminescence on said carrier is provided outside of said carrier housing section.

11. A carrier housing/processing apparatus according to claim 10, wherein in said carrier housing section, a side face provided with said measuring equipment is formed in a plane.

12. A carrier housing/processing apparatus according to any one of claim 1 through claim 11, wherein said carrier contains a magnetic substance and said carrier is held in a predetermined position of said carrier housing section due to a magnetic field exerted from outside of said carrier housing section.

13. A carrier housing/processing apparatus according to any one of claim 1 through claim 12, wherein said carrier is a glass or its surface is coated with a glass.

14. A carrier housing/processing method comprising;
a drawing/contacting step for, with respect to a housing section which accommodates one or a plurality of carriers fixed or able to be fixed with chemical substances such as ligands and has an inlet/outlet which enables a fluid to pass through but does not enable said carriers to pass through, drawing fluid through said inlet/outlet from external containers by a drawing/discharging section, to contact said carriers which are held in said carrier housing section by self-weight of said carrier, frictional force with the inner wall of said carrier housing section, or remote force from outside with respect to said carrier, with the drawn fluid; and
a discharging step for discharging only said fluid through said inlet/outlet by said drawing/discharging section, in a state where said carrier is accommodated in said carrier housing section.

15. A carrier housing/processing method according to claim 14, further comprising a transferring step for transferring said inlet/outlet relatively with respect to containers provided outside.

16. A carrier housing/processing method according to either one of claim 14 and claim 15, wherein said reaction step has a step for repeatedly drawing and discharging a fluid with respect to said carrier housing section.

17. A carrier housing/processing method according to any one ofclaim 14 through claim 16, further comprising an accommodating step for accommodating said carrier in said carrier housing section from an opening having a size enabling said carrier to pass through.

18. A carrier housing/processing method according to anyone of claim 14 through claim 17, further comprising a removing step for removing said carrier from said carrier housing section through an opening having a size enabling said carrier provided in said carrier housing section to pass through.
